# EUROPÄISCHE PATENTSCHRIFT

(11) **EP 3 376 966 B1**
(45) Veröffentlichungstag und Bekanntmachung des Hinweises auf die Patenterteilung: **16.10.2019**
(21) Anmeldenummer: 16805716.4
(22) Anmeldetag: 17.11.2016
(51) Int. Cl.: A61B 17/00, A61B 17/04, A61B 17/06, A61B 17/062

(54) **MEDIZINISCHES INSTRUMENT**
MEDICAL INSTRUMENT
INSTRUMENT MÉDICAL

(30) Priorität: 20.11.2015 AT 509912015
(43) Veröffentlichungstag der Anmeldung: 26.09.2018
(73) Patentinhaber: Klaffenböck, Johann, 5350 Strobl (AT); Klaffenböck, Lukas, 5350 Strobl (AT); Mair, Julian, 81925 München (DE)
(72) Erfinder: Klaffenböck, Johann, 5350 Strobl (AT); Klaffenböck, Lukas, 5350 Strobl (AT); Mair, Julian, 81925 München (DE)
(74) Vertreter: Babeluk, Michael
(86) Internationale Anmeldenummer: PCT/AT2016/060111
(87) Internationale Veröffentlichungsnummer: WO 2017/083897

(56) Entgegenhaltungen:
- EP-A1- 2 429 377
- WO-A2-2008/045333
- DE-A1-102012 025 210
- US-A1- 2014 257 345

## Beschreibung

Die Erfindung betrifft eine Vorrichtung für ein medizinisches Instrument zum Anbringen einer chirurgischen Naht, mit einer Auslöseeinheit und einer Operationseinheit, wobei die Operationseinheit zwei verschwenkbare Greifbacken aufweist, wobei jede Greifbacke eine Nadelaufnahme sowie eine Nadelfixiereinrichtung aufweist, und wobei die Auslöseeinheit zur Bewegung der beiden Greifbacken eingerichtet ist, wobei die Nadelfixiereinrichtung über zumindest ein Klinkenelement verfügt, das über die Auslöseeinheit bewegbar ist, und das zumindest eine Klinkenelement mit einem Zahnradelement zusammenwirkt, sowie die Verwendung dieser Vorrichtung in einem chirurgischen Instrument.

Bei zahlreichen chirurgischen Eingriffen ist es notwendig, eine chirurgische Naht zu setzen. Hierbei haben sich die Operationsmethoden aufgrund neuartiger Instrumente derart verbessert, dass zahlreiche Eingriffe endoskopisch durchgeführt werden können, um auf diese Weise die Belastung des Patienten minimieren zu können. So beschreibt die EP 2 083 727 B1 eine Vorrichtung der eingangs erwähnten Art, wobei ein zangenartiger Endeffektor dazu eingerichtet ist, eine chirurgische Naht zu setzen. Ähnliche Vorrichtungen können auch der EP 2 462 877 A2 sowie der WO 2008/045333 A2 entnommen werden.

Die in dem Stand der Technik beschriebenen Vorrichtungen zum Setzen einer chirurgischen Naht sind häufig äußerst kompliziert aufgebaut und benötigen zwei unabhängige Betätigungssysteme, nämlich ein erstes zur Betätigung der Greifbacken und ein zweites zur Fixierung der Nadel in der jeweiligen Greifbacke beziehungsweise für die Nadelübergabe zwischen den beiden Greifbacken.

In der US 5,632,751 A ist eine chirurgische Nähvorrichtung offenbart, wobei zwei Nadelhalteeinrichtungen vorgesehen sind, die jeweils entweder die Nadelspitze oder das Nadelende mit dem Nadelöhr fixieren. Die Handhabung dieser Vorrichtung während des Setzens der Naht ist kompliziert, weil es vor jedem Nadelstich in seiner Längsachse um 180° verdreht werden muss. Dabei besteht die Gefahr, dass sich das Nahtmaterial in der Vorrichtung verheddert.

Die US 2014/0257345 A1 offenbart eine chirurgische Näheinrichtung der eingangs erwähnten Art, wobei die Auslöseeinheit ein Klinkenelement bewegt, das mit einem Zahnrad zur Fixierung der Nadel in einer Greifbacke zusammenwirkt.

Es ist Aufgabe der Erfindung, die bekannt gewordenen Vorrichtungen weiterzubilden, und insbesondere eine Vorrichtung zur Verfügung zu stellen, die einfach zu bedienen ist und eine stabile und korrekt gesetzte chirurgische Naht liefert.

Diese Aufgabe wird durch die eingangs erwähnte Vorrichtung erfindungsgemäß dadurch gelöst, dass das Zahnradelement zwei Zahnräder aufweist, die im Wesentlichen planparallel zueinander angeordnet sind und eine unterschiedliche Anzahl an Zähnen aufweisen. Die Erfindung hat den Vorteil, dass ein einziges Auslösesystem sowohl für die Bewegung der Greifbacken als auch die Nadelübergabe zuständig ist. Durch die unterschiedliche Zahnanzahl der beiden Zahnräder sind unterschiedliche Positionen der Greifbacken zueinander und/oder der zumindest einen Nahtnadel einstellbar. Dies erlaubt eine einfache Bedienung sowie eine schlanke Bauweise, die insbesondere bei endoskopischen Operationsmethoden von Vorteil ist.

In diesem Zusammenhang wird darauf hingewiesen, dass in der vorliegenden Offenbarung unter "Operationseinheit" jener Teil der erfindungsgemäßen Vorrichtung verstanden wird, der die gestellte Aufgabe, nämlich das Setzen einer chirurgischen Naht, unmittelbar erfüllt, während die "Auslöseeinheit" die Bewegungen und Funktionen der Operationseinheit steuert.

Erfindungsgemäß ist vorgesehen, dass die Auslöseeinheit zusätzlich zur Betätigung der Nadelfixiereinrichtung eingerichtet ist. In einer allgemeinen Ausführung der Erfindung kann dies beispielsweise derart realisiert sein, dass die Auslöseeinheit in einem ersten Hub die Greifbacken schließt, und in einem zweiten, daran anschließenden Hub die Nadel in einer Greifbacke fixiert. Dabei kann über ein externes Signal festgelegt werden, in welcher der beiden Greifbacken die Nadel fixiert wird. Dies kann beispielsweise durch ein elektrisch angesteuertes Umschaltorgan realisiert werden.

Besonders bevorzugt ist jedoch eine besondere Ausführungsvariante der vorliegenden Erfindung, bei der die Umschaltung vollautomatisch erfolgt. Hierbei fixiert die Auslöseeinheit beispielsweise im zweiten Hub einer ersten Betätigung die Nadel an einer ersten Greifbacke, und in einem daran anschließenden zweiten Hub einer zweiten Betätigung die Nadel an der zweiten Greifbacke. Nach der ersten Betätigung erfolgt ein Zwischenschritt, bei welchem die beiden Greifbacken auseinander bewegt werden, bevor die zweite Betätigung zur Fixierung der Nadel in der zweiten Greifbacke, also die Nadelübergabe erfolgt.

Vorzugsweise sind die Greifbacken gelenkig miteinander verbunden und können so bei der Betätigung zueinander geschwenkt oder auseinander bewegt werden. Diese gelenkige Verbindung ist in einer besonders bevorzugten Ausführung derart realisiert, dass die beiden Greifbacken jeweils über eine kulissenartige Ausnehmung mit einem ersten Ende und einem zweiten Ende verfügen, in die ein Auslöseelement der Auslöseeinheit, vorzugsweise ein Kulissenschieber angeordnet ist. Bei Auslösung einer Bewegung über die Auslöseeinheit wird das Verschwenken der Greifbacken verursacht. Hierbei wird der Kulissenschieber innerhalb der kulissenartigen Ausnehmung verfahren, wobei der gekrümmte Verlauf der kulissenartigen Ausnehmung das Einnehmen von definierten Betriebszuständen erlaubt, die an anderer Stelle noch im Detail beschrieben werden.

Eine besonders einfache und sichere Betätigung der erfindungsgemäßen Vorrichtung wird ermöglicht, wenn die Auslöseeinheit über ein Hydrauliksystem verfügt. Ein hierfür geeignetes Hydrauliksystem kann beispielsweise der EP 2 429 377 B1 der Anmelder entnommen werden.

Eine präzise Übergabe und Fixierung der Nadel in der jeweiligen Greifbacke erhalten, wenn die Nadelfixiereinrichtung über zumindest ein Klinkenelement verfügt, das über die Auslöseeinheit bewegbar ist. Das zumindest eine Klinkenelement wirkt hierbei mit einem Zahnradelement zusammen.

Die Begriffe "Zahnrad" und "Zahnradelement" sind hier nicht auf Maschinenelemente beschränkt, die mit anderen Zahnrädern oder Zahnstangen kämmen. Erfindungsgemäß wirkt ein erstes Zahnrad mit einer Sperrklinke zusammen, die bei entsprechender Betätigung das Zahnrad durch eine Bewegung in ihrer Längsrichtung antreibt. Das zweite Zahnrad entspricht etwa einem Rad mit mehreren am Umfang angeordneten Nocken, die eine Stoßstange in ihrer Axialrichtung verschieben. Besonders bevorzugt ist hierbei vorgesehen, dass das zweite Zahnrad doppelt so viele Zähne wie das erste Zahnrad aufweist.

Hierbei weist das Zahnradelement zwei Zahnräder auf, die planparallel zueinander angeordnet sind und eine unterschiedliche Anzahl von Zähnen aufweisen. Diese zwei Zahnräder sind derart miteinander verbunden, dass, sobald ein erstes Zahnrad verdreht wird, das zweite Zahnrad ebenfalls verdreht wird. Besonders bevorzugt ist hierbei vorgesehen, dass das Zahnradelement einstückig gefertigt ist.

Bei Verschieben des Klinkenelements entlang der Greifbacke wird das erste Zahnrad des Zahnradelements verdreht. Da die beiden Zahnräder des Zahnradelements miteinander verbunden sind, verdreht sich bei Bewegung des Klinkenelements auch das zweite Zahnradelement. Durch die unterschiedliche Zahnanzahl der beiden Zahnräder ergibt sich eine Veränderung der Betätigungsfrequenz, wodurch unterschiedliche Positionen der Greifbacken zueinander und/oder der zumindest einen Nahtnadel einstellbar sind.

Besonders bevorzugt ist des Weiteren vorgesehen, dass die Nadelfixiereinrichtung über zumindest ein bewegbares Schieberelement verfügt, wobei das zumindest eine Schieberelement dazu eingerichtet ist, zumindest eine Nahtnadel in der Nadelaufnahme zu fixieren. Hierbei wirkt das zumindest eine Schieberelement mit dem zumindest einen Zahnradelement, insbesondere mit dem zweiten Zahnrad zusammen, während wie bereits zuvor beschrieben das Klinkenelement mit dem ersten Zahnrad in Eingriff steht.

Die Bewegung des Schieberelements zur Fixierung der zumindest einen chirurgischen Nahtnadel und insbesondere das Lösen der Fixierung der zumindest einen chirurgischen Nahtnadel wird in einer weiteren Ausführung der Erfindung dadurch unterstützt, dass zumindest ein erstes Federelement mit dem zumindest einen Schieberelement zusammenwirkt.

Alternativ dazu ist grundsätzlich auch eine Zwangsführung möglich, bei der die Bewegung des Schieberelements in beide Richtungen zwangsläufig durch das zweite Zahnrad bewirkt wird. In diesem Fall umgreift das Schiebeelement beispielsweise das Zahnrad und es wird kein Federelement benötigt.

Eine weitere Verbesserung der Fixierung der zumindest einen Nahtnadel wird erreicht, wenn zumindest ein weiteres Federelement mit dem zumindest einen Klinkenelement zusammenwirkt.

Die erfindungsgemäße Vorrichtung hat sich insbesondere bei der Verwendung in einem chirurgischen Instrument, insbesondere einem flexiblen endoskopischen Instrument, das vorzugsweise für die transorale Platzierung in einem Magen konfiguriert ist, bewährt. Hierbei ist an dem proximalen Ende des länglichen Elements des chirurgischen Instruments zum Anbringen einer chirurgischen Naht eine Betätigungseinrichtung angeordnet, wobei die erfindungsgemäße Vorrichtung an dem distalen Ende des länglichen Elements angeordnet ist und mit der Betätigungseinrichtung zusammenwirkt.

Im Folgenden wird anhand von nichteinschränkenden Ausführungsbeispielen mit zugehörigen Figuren die Erfindung näher erläutert. Darin zeigen:
- Figuren 1A und 1B:: zwei Seitenansichten der erfindungsgemäßen Vorrichtung in einer ersten, geöffneten Position;
- Figuren 2A und 2B:: zwei Seitenansichten der Vorrichtung aus den Figuren 1A und 1B in einer ersten geschlossenen Position;
- Figuren 3A und 3B:: zwei Seitenansichten der Vorrichtung aus den Figuren 1A und 1B in einer zweiten geschlossenen Position;
- Figuren 4A und 4B:: zwei Seitenansichten der Vorrichtung aus den Figuren 1A und 1B in einer dritten geschlossenen Position;
- Figur 5:: eine erste Ausführung der erfindungsgemäßen Vorrichtung in einer perspektivischen Ansicht;
- Figuren 6A und 6B:: die Operationseinheit der Vorrichtung aus Figur 5;
- Figur 7:: die Operationseinheit im geöffneten Zustand;
- Figur 8A:: eine erste Seitenansicht des erfindungsgemäßen Zahnradelements; und
- Figur 8B:: eine zweite Seitenansicht des erfindungsgemäßen Zahnradelements.

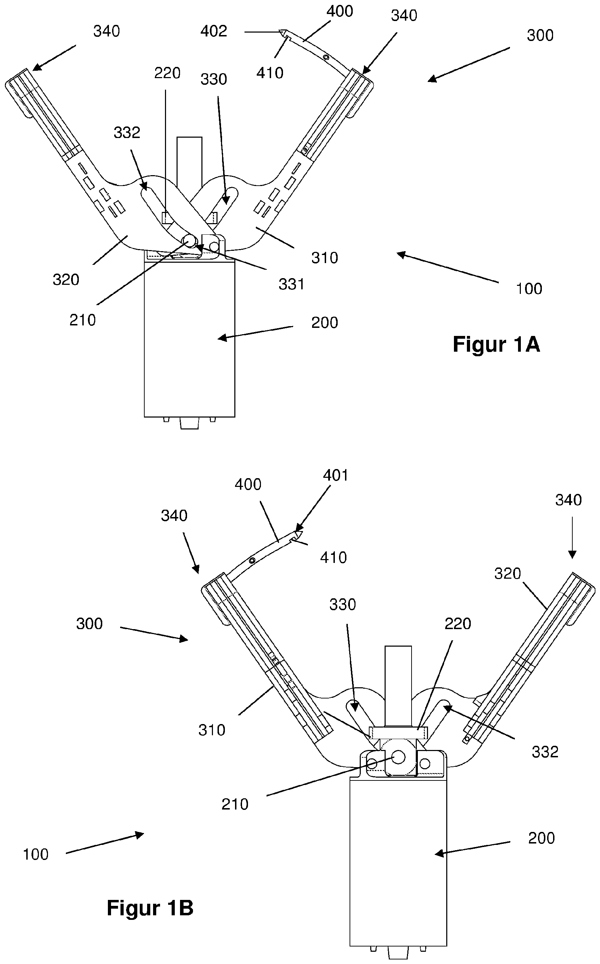

In den Figuren 1A und 1B ist die erfindungsgemäße Vorrichtung 100 in einer ersten geöffneten Position dargestellt. Diese Vorrichtung 100 wird üblicherweise als Endeffektor bezeichnet, wobei im Rahmen dieser Offenbarung der Begriff "Endeffektor" synonym für die erfindungsgemäße Vorrichtung zum Anbringen einer chirurgischen Naht verwendet wird.

Der Endeffektor 100 weist eine Auslöseeinheit 200 sowie eine Operationseinheit 300 auf. Die Auslöseeinheit 200 ist bevorzugterweise als hydraulisches Kolbensystem (nicht dargestellt) ausgebildet.

Die Operationseinheit 300, die zum Setzen der chirurgischen Naht verantwortlich ist, weist zwei Greifbacken 310, 320 auf, die in dieser Ausführung der Erfindung im Wesentlichen spiegelbildlich aufgebaut sind. Die Operationseinheit 300 wird von der Auslöseeinheit 200 bedient, was sowohl die Bewegung der beiden Greifbacken 310, 320 als auch die Fixierung der Nahtnadel 400 in der Greifbacke 310, 320 betrifft. Die Auslöseeinheit 200 selbst wird wiederum von einem Betätigungselement eines chirurgischen Instruments angesteuert (nicht dargestellt). Zu Anschauungszwecken ist das im Gebrauch angebrachte äußere Gehäuse des Endeffektors 100 entfernt.

Die Greifbacken 310, 320 weisen jeweils eine kulissenartige Ausnehmung 330 auf, wobei die Greifbacken 310, 320 derart zueinander angeordnet sind, dass in die Ausnehmungen 330 ein Kulissenschieber 210 der Auslöseeinheit 220 eingreift. Bei dem in den Figuren 1A und 1B dargestellten offenen Zustand des Endeffektors 100 befindet sich der Kulissenschieber 210 an einem ersten Ende 331 der Ausnehmung 330. Die Auslöseeinheit 200 weist des Weiteren eine Anschlagplatte 220 (Figur 1B) auf, die in kraftschlüssiger Verbindung mit dem Kulissenschieber 210 steht. In dieser Position ist eine Nahtnadel 400 mit ihrem ersten Ende 401 in der Nadelaufnahme 340 der ersten Greifbacke 310 fixiert. Die Nahtnadel 400 weist hierfür eine nutartige Nadelausnehmung 410 auf, die der Fixierung der Nahtnadel 400 in einer Nadelaufnahme 340 dient.

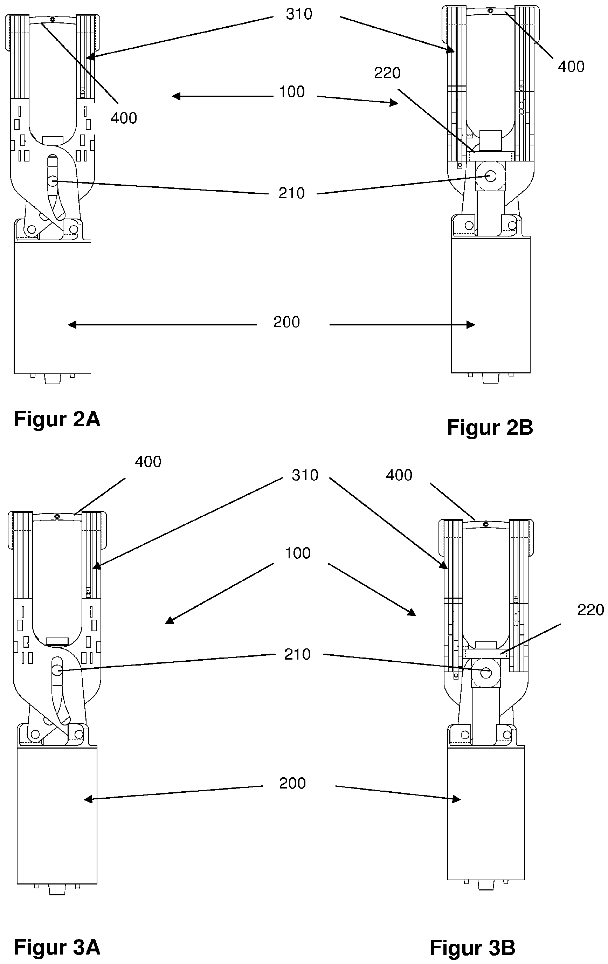

Bei Betätigung des Kulissenschiebers 210 bewegt sich dieser in der Ausnehmung 330 von dem ersten Ende 331 in Richtung des zweiten Endes 332. Bei Betätigung der Auslöseeinheit 200 wird der Kulissenschieber 210 in eine zweite Position verschoben, wodurch die beiden Greifbacken 310, 320 zueinander bewegt werden, und das zweite Nadelende 402 der Nahtnadel 400 in die Nadelaufnahme 340 der zweiten Greifbacke 320 eingeführt wird (Figuren 2A und 2B).

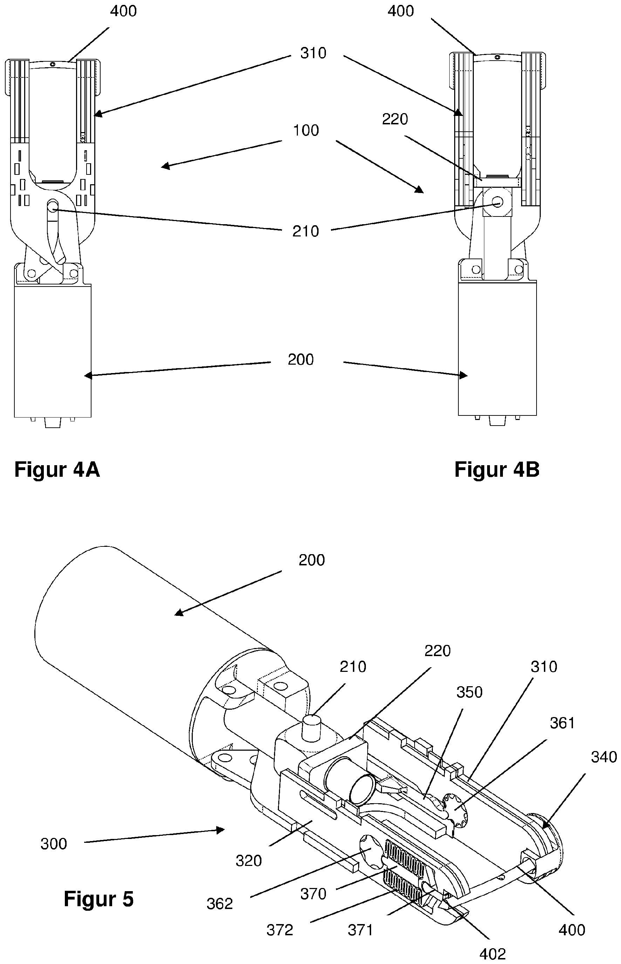

Wird der Kulissenschieber 210 weiter in Richtung zweites Ende 332 der kulissenartigen Ausnehmung 330 über die Auslöseeinheit 200 bewegt, wie insbesondere in den Figuren 3A und 3B sowie 4A und 4B gezeigt, so verändert sich die Position der Greifbacken 310, 320 zueinander nicht mehr. Die Bewegung des Kulissenschiebers 210 über die in den Figuren 2A und 2B gezeigte Position hinaus bewirkt nun eine Auslösung der beiden Nadelfixiereinheiten, die jeweils innerhalb der Greifbacken 310, 320 angeordnet sind und insbesondere in den Figuren 5 bis 9 nachfolgend näher beschrieben werden.

In der Figur 5 ist der erfindungsgemäße Endeffektor 100 in jener Position dargestellt, die der ersten geschlossenen Position gemäß den Figuren 2A und 2B entspricht. Die Nahtnadel 400 ist hier in der Nadelaufnahme 340 der ersten Greifbacke 310 fixiert. Die Anschlagplatte 220 steht im direkten Kontakt mit einem Klinkenelement 350, das über eine Rastnase 351 mithilfe der Anschlagplatte 220 im Wesentlichen entlang der Längsachse der ersten Greifbacke 310 verschoben werden kann.

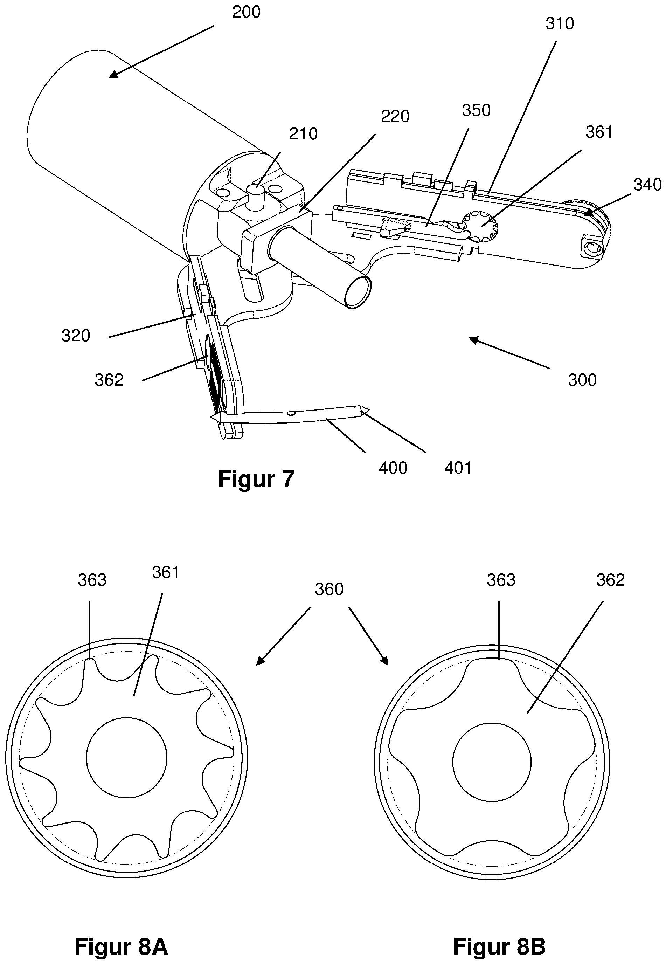

Das Klinkenelement 350 greift hierbei in ein erstes Zahnrad 361 eines Zahnradelementes 360 ein, das im Detail in den Figuren 8A und 8B dargestellt ist. Das Zahnradelement 360 besteht aus zwei planparallel zueinander angeordneten Zahnrädern 361, 362, die eine unterschiedliche Anzahl an Zähnen 363 aufweisen. Wird nun das erste Zahnrad 361 verdreht, verdreht sich aufgrund der gleichen Drehachse auch das zweite Zahnrad 362. Im vorliegenden Beispiel verfügt das erste Zahnrad 361 über zehn Zähne 363, während das zweite Zahnrad 362 des Zahnradelements 360 fünf Zähne 363 aufweist. Somit verfügt dieses Zahnradelement 360 über ein Verhältnis der Betätigungsfrequenz von 1:2. Zudem ist das erste Zahnrad 361 als Klinkenrad ausgeführt.

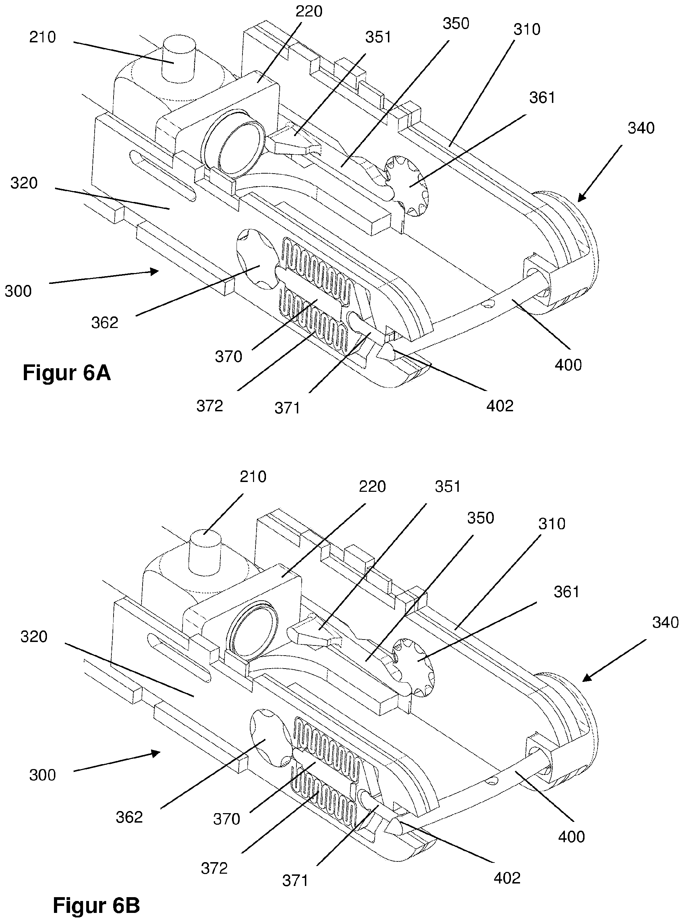

Wird die Anschlagplatte 210 in Richtung Nadel 400 bewegt, so wird auch das Klinkenelement 350 in diese Richtung bewegt, wodurch das erste Zahnrad 361 des Zahnradelements 360 gemäß der Darstellung in Figur 6A entgegen den Uhrzeigersinn verdreht wird. Durch die Verdrehung des ersten Zahnrades 361 wird gleichzeitig in die gleiche Richtung auch das mit dem ersten Zahnrad 361 verdrehfest verbundene zweite Zahnrad 362 verdreht, wobei aufgrund der ungleichen Anzahl an Zähnen 363 des ersten Zahnrades 361 und des zweiten Zahnrades 362 erst mit ausreichendem Vorschub der Anschlagplatte 210 eine Bewegung eines Schieberelements 370 über das zweite Zahnrad 362 ausgelöst wird, wodurch wiederum eine Fixierung oder ein Lösen eines Endes 401 der Nahtnadel 400 erfolgt.

Das Schieberelement 370 greift in das zweite Zahnrad 362 ein, wie insbesondere in der Figur 5 im Bereich der zweiten Greifbacke 320 gezeigt ist. Dieses Schieberelement 370 weist an seinem dem zweiten Zahnrad 362 abgewandten Ende einen Stößel 371 auf, der dazu eingerichtet ist, in die nutartige Nadelausnehmung 410 der Nahtnadel 400 einzugreifen, um auf diese Weise das Ende 401, 402 der Nahtnadel 400 in der Nadelaufnahme 340 zu fixieren.

In der Figur 6A liegt das zweite Ende 402 der Nahtnadel 400 noch unfixiert in der Nadelaufnahme 340 der zweiten Greifbacke 320, wobei aufgrund eines weiteren Vorschubs des Kulissenschiebers 210 eine Verdrehung des zweiten Zahnrades 362 in der zweiten Greifbacke 320 dazu führt, dass der Stößel 371 des Schieberelements 370 in der zweiten Greifbacke 320 derart verschoben wird, dass er in die nutartige Nadelausnehmung 410 der Nahtnadel 400 eingreift, und so das zweite Ende 402 der Nahtnadel 400 in der Nadelaufnahme 340 fixiert.

Bei weiterem Vorschub der Anschlagplatte 210 führt die Verdrehung des Zahnradelements 360 der ersten Greifbacke 310 dazu, dass mithilfe eines ersten Federelements 372 der Stößel 371 zurückgezogen wird und das erste Nadelende 401 der Nahtnadel 400 in der Nadelaufnahme 340 der ersten Greifbacke 310 freigibt.

Wird nun der Endeffektor 100 durch Auseinanderbewegen der Greifbacken 310, 320 wieder geöffnet (Figur 7), ist jetzt die Nahtnadel 400 in der zweiten Greifbacke 320 fixiert. Durch neuerliches Schließen des Endeffektors 100 bis zur Endposition gemäß den Figuren 4A und 4B wird die Nahtnadel 400 wiederum in der ersten Greifbacke 310 angeordnet und fixiert. Somit kann durch abwechselndes Öffnen und Schließen des Endeffektors 100 die Nadel 400 zwischen den beiden Greifbacken 310, 320 hin und her bewegt werden, um auf diese Weise eine Nähbewegung auszuführen und damit eine chirurgische Naht herzustellen.

Es versteht sich, dass die vorliegende Erfindung nicht auf die dargestellten Ausführungsbeispiele beschränkt ist. Insbesondere kann die Form der Greifbacke und/ oder der Nahtnadel unterschiedlich ausgeführt sein, auch ist es nicht zwingend notwendig, dass die Bewegung der Greifbacke und/oder des Schieberelements und/oder des Klinkenelements mithilfe eines Hydrauliksystems ausgelöst wird. So wird beispielsweise in einer weiteren Ausführung der Erfindung die Fixierung beziehungsweise das Lösen der jeweiligen Enden 401, 402 der Nahtnadel 400 dadurch weiter verbessert, dass zusätzliche Federelemente zur Stabilisierung des Stößels 371 vorgesehen sind. Diese zusätzlichen Federelemente sorgen für einen konstanten Anpressdruck des Stößels 371 in die nutartige Nadelausnehmung 410. Weitere Federelemente können des Weiteren für die Stabilisierung und Bewegung des Klinkenelements 350 vorgesehen sein.

## Patentansprüche

1. Vorrichtung (100) für ein medizinisches Instrument zum Anbringen einer chirurgischen Naht, mit einer Auslöseeinheit (200) und einer Operationseinheit (300), wobei die Operationseinheit (300) zwei verschwenkbare Greifbacken (310, 320) aufweist, wobei jede Greifbacke (310, 320) eine Nadelaufnahme (340) sowie eine Nadelfixiereinrichtung aufweist, wobei die Auslöseeinheit (200) zur Bewegung der beiden Greifbacken (310, 320) sowie zusätzlich zur Betätigung der Nadelfixiereinrichtung eingerichtet ist, und wobei die Nadelfixiereinrichtung über zumindest ein Klinkenelement (350) verfügt, das über die Auslöseeinheit (200) bewegbar ist, wobei das zumindest eine Klinkenelement (350) mit einem Zahnradelement (360) zusammenwirkt, **dadurch gekennzeichnet, dass** das Zahnradelement (360) zwei Zahnräder (361, 362) aufweist, die im Wesentlichen planparallel zueinander angeordnet sind und eine unterschiedliche Anzahl an Zähnen (363) aufweisen.

2. Vorrichtung (100) nach Anspruch 1, **dadurch gekennzeichnet, dass** die beiden Greifbacken (310, 320) jeweils über eine kulissenartige Ausnehmung (330) mit einem ersten Ende (331) und einem zweiten Ende (332) verfügen, in die ein Auslöseelement der Auslöseeinheit (200), vorzugsweise ein Kulissenschieber (210), angeordnet ist.

3. Vorrichtung (100) nach Anspruch 1 oder 2, **dadurch gekennzeichnet,** die Auslöseeinheit (200) über ein Hydrauliksystem verfügt.

4. Vorrichtung (100) nach einem der Ansprüche 1 bis 3, **dadurch gekennzeichnet, dass** die Nadelfixiereinrichtung über zumindest ein bewegbares Schieberelement (370) verfügt, wobei das zumindest eine Schieberelement (370) dazu eingerichtet ist, zumindest eine Nahtnadel (400) in der Nadelaufnahme (340) zu fixieren.

5. Vorrichtung (100) nach Anspruch 4, **dadurch gekennzeichnet, dass** das zumindest eine Schieberelement (370) mit dem zumindest einen Zahnradelement (360) zusammenwirkt.

6. Vorrichtung (100) nach Anspruch 4 oder 5, **dadurch gekennzeichnet, dass** zumindest ein erstes Federelement (372) mit dem zumindest einen Schieberelement (370) zusammenwirkt, und vorzugsweise zumindest ein weiteres Federelement mit dem zumindest einen Klinkenelement (350) zusammenwirkt.

7. Chirurgisches Instrument zum Anbringen einer chirurgischen Naht mit einem länglichen Element, an dessen proximalen Ende eine Betätigungseinrichtung angeordnet ist, **dadurch gekennzeichnet, dass** eine Vorrichtung (100) nach einem der Ansprüche 1 bis 6 an seinem distalen Ende angeordnet ist und mit der Betätigungseinrichtung zusammenwirkt.

8. Chirurgisches Instrument nach Anspruch 7, **dadurch gekennzeichnet, dass** die beiden Greifbacken (310, 320) der Operationseinheit (300) aus einer Offenposition in eine Schließposition bewegbar sind, wobei in der Offenposition die Nahtnadel (400) mit ihren ersten Nadelende (401) in der Nadelaufnahme (340) der ersten Greifbacke (310) fixiert ist, und bei Betätigung der Auslöseeinheit (200) durch die Betätigungseinrichtung ein Auslöseelement, insbesondere ein Kulissenschieber (210) entlang der kulissenartigen Ausnehmung (330) bewegbar ist, wodurch die beiden Greifbacken in die Schließposition überführbar sind, und gleichzeitig das zweite Nadelende (402) der Nahtnadel (400) in die Nadelaufnahme (340) der zweiten Greifbacke (320) fixierbar ist, während das erste Nadelende (401) der Nahtnadel (400) in der Nadelaufnahme (340) der ersten Greifbacke (320) freigegeben ist.

## Claims

1. Device (100) for a medical instrument for applying a surgical suture, comprising a release unit (200) and an operating unit (300), wherein the operating unit (300) has two pivotable gripper jaws (310, 320), wherein each gripper jaw (310, 320) comprises a needle receptacle (340) and a needle-fixing device, wherein the release unit (200) is adapted for moving the two gripper jaws (310, 320) and additionally for actuating the needle-fixing device, and wherein the needle-fixing device has at least one pawl element (350) which is movable via the release unit (200), wherein the at least one pawl element (350) cooperates with a gear element (360), **characterised in that** the gear element (360) has two gears (361, 362), which are arranged substantially plane-parallel to one another and have a different number of teeth (363).

2. Device (100) according to claim 1, **characterised in that** the two gripper jaws (310, 320) each have a slot-like recess (330) having a first end (331) and a second end (332), in which a release element of the release unit (200) is arranged, preferably a sliding block slide (210).

3. Device (100) according to claim 1 or 2, **characterised in that** the release unit (200) has a hydraulic system.

4. Device (100) according to one of the claims 1 to 3, **characterised in that** the needle-fixing device has at least one movable slide element (370), wherein the at least one slide element (370) is adapted to fix at least one suture needle (400) in the needle receptacle (340).

5. Device (100) according to claim 4, **characterised in that** the at least one slide element (370) cooperates with the at least one gear element (360).

6. Device (100) according to claim 4 or 5, **characterised in that** at least one first spring element (372) cooperates with the at least one slide element (370), and preferably at least one further spring element cooperates with the at least one pawl element (350).

7. Surgical instrument for applying a surgical suture with an elongated element, at the proximal end of which an actuating device is arranged, **characterised in that** a device (100) according to one of the claims 1 to 6 is arranged at its distal end and cooperates with the actuating device.

8. Surgical instrument according to claim 7, **characterised in that** the two gripper jaws (310, 320) of the operating unit (300) are movable from an open position to a closed position, wherein in the open position the suture needle (400) is fixed with its first needle end (401) in the needle receptacle (340) of the first gripper jaw (310), and upon actuation of the release unit (200) by the actuating device, a release element, in particular a sliding block slide (210), is movable along the slot-like recess (330), whereby the two gripper jaws can be transferred to the closed position, and at the same time the second needle end (402) of the suture needle (400) is fixable in the needle receptacle (340) of the second gripper jaw (320), while the first needle end (401) of the suture needle (400) is released in the needle receptacle (340) of the first gripper jaw (320).

## Revendications

1. Dispositif (100) destiné à un instrument médical permettant d'effectuer une suture chirurgicale, comprenant une unité de déclenchement (200) et une unité d'opération (300), l'unité d'opération (300) comprenant deux mâchoires de préhension pivotantes (310, 320), chaque mâchoire de préhension (310, 320) comprenant un logement de réception d'aiguille (340) ainsi qu'un dispositif de fixation d'aiguille, l'unité de déclenchement (200) étant réalisée pour permettre de déplacer les deux mâchoires de préhension (310, 320) ainsi qu'en outre d'actionner le dispositif de fixation d'aiguille, et le dispositif de fixation d'aiguille étant équipé d'au moins un élément de cliquet (350) pouvant être déplacé par l'intermédiaire de l'unité de déclenchement (200), l'élément de cliquet (350) coopérant avec un élément de roue dentée (360),
**caractérisé en ce que**
l'élément de roue dentée (360) comporte deux roues dentées (361, 362) qui sont montées selon une orientation essentiellement parallèle, plane l'une par rapport à l'autre et comportent un nombre de dents différent (363).

2. Dispositif (100) conforme à la revendication 1,
**caractérisé en ce que**
les deux mâchoires de préhension (310, 320) sont équipées chacune d'un évidement en forme de coulisse (330) ayant une première extrémité (331) et une seconde extrémité (332) dans lequel est positionné un élément de déclenchement de l'unité de déclenchement (200), de préférence un curseur (210).

3. Dispositif (100) conforme à la revendication 1 ou 2,
**caractérisé en ce que**
l'unité de déclenchement (200) est équipée d'un système hydraulique.

4. Dispositif (100) conforme à l'une des revendications 1 à 3,
**caractérisé en ce que**
le dispositif de fixation d'aiguille est équipé d'au moins un élément coulissant mobile (370), cet élément coulissant mobile (370) étant réalisé pour permettre de fixer au moins une aiguille de suture (400) dans le logement de réception d'aiguille (340).

5. Dispositif (100) conforme à la revendication 4,
**caractérisé en ce que**
l'élément coulissant (370) coopère avec l'élément à roue dentée (360).

6. Dispositif (100) conforme à la revendication 4 ou 5,
**caractérisé en ce qu'**
au moins un premier élément de ressort (372) coopère avec l'élément coulissant (370) et de préférence au moins un autre élément de ressort coopère avec l'élément de cliquet (350).

7. Instrument chirurgical permettant d'effectuer une suture chirurgicale comprenant un élément longitudinal à l'extrémité proximale duquel est positionné un dispositif d'actionnement,
**caractérisé en ce qu'**
un dispositif (100) conforme l'une des revendications 1 à 6, est positionné à son extrémité distale et coopère avec le dispositif d'actionnement.

8. Instrument chirurgical conforme à la revendication 7,
**caractérisé en ce que**
les deux mâchoires de préhension (310, 320) de l'unité d'opération (300) peuvent être déplacées entre une position ouverte et une position fermée, et dans la position fermée, l'aiguille de suture (400) est située par sa première extrémité d'aiguille (401) dans le logement de réception d'aiguille (340) de la première mâchoire de préhension (310), et lors d'un actionnement de l'unité de déclenchement (200) par l'unité d'actionnement, un élément de déclenchement, en particulier un curseur (210) peut être déplacé le long de l'évidement en forme de coulisse (330), de sorte que les deux mâchoires de préhension soient transférées dans la position de fermeture, et que simultanément la seconde extrémité d'aiguille (402) de l'aiguille de suture (400) puisse être fixée dans le logement de réception d'aiguille (340) de la seconde mâchoire de préhension (320), alors que la première extrémité d'aiguille (401) de l'aiguille de suture (400) est libérée dans le logement de réception d'aiguille (340) de la première mâchoire de préhension (320).
